(19) Europäisches Patentamt
European Patent Office
Office européen des brevets

(11) **EP 2 855 018 B1**

(12) **FASCICULE DE BREVET EUROPEEN**

(45) Date de publication et mention de la délivrance du brevet:
**18.10.2017 Bulletin 2017/42**

(21) Numéro de dépôt: **13720986.2**

(22) Date de dépôt: **05.04.2013**

(51) Int Cl.:
*B01J 37/02* (2006.01)   *B01J 23/44* (2006.01)
*B01J 23/755* (2006.01)   *B01J 37/16* (2006.01)
*B01J 37/20* (2006.01)   *C10G 45/40* (2006.01)
*C10G 45/36* (2006.01)

(86) Numéro de dépôt international:
**PCT/FR2013/050755**

(87) Numéro de publication internationale:
**WO 2013/175085 (28.11.2013 Gazette 2013/48)**

(54) **PROCEDE DE PREPARATION D'UN CATALYSEUR A BASE D'UN METAL DU GROUPE VIII ET CONTENANT DU SILICIUM ET PROCEDE D'HYDROGENATION SELECTIVE METTANT EN OEUVRE LEDIT CATALYSEUR**

PROZESS ZUR HERSTELLUNG EINES KATALYSATORS ENTHALTEND EIN METALL DER VIII NEBENGRUPPE UND SILIZIUM UND SELEKTIVE HYDRIERUNG IN GEGENWART DIESES KATALYSATORS

PROCESS OF PREPARATION OF A CATALYST COMPRISING A GROUP VIII METAL AND SILICON AND PROCESS OF SELECTIVE HYDROGENATION USING THAT CATALYST

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.05.2012 FR 1201492**

(43) Date de publication de la demande:
**08.04.2015 Bulletin 2015/15**

(73) Titulaire: **IFP Énergies nouvelles
92852 Rueil-Malmaison Cedex (FR)**

(72) Inventeurs:
• **DUBREUIL, Anne-Claire**
  **F-69003 Lyon (FR)**
• **MARQUES MOTA, Filipe Manuel**
  **F-69003 Lyon (FR)**
• **JANVIER, Josselin**
  **F-92000 Nanterre (FR)**

(56) Documents cités:
WO-A1-2012/029714   DE-A1- 19 757 990
US-A1- 2009 143 491   US-A1- 2012 071 700

• QUINTANILLA A ET AL: "Tuning the support adsorption properties of Pd/SiO2 by silylation to improve the selective hydrogenation of aromatic ketones", JOURNAL OF CATALYSIS, ACADEMIC PRESS, DULUTH, MN, US, vol. 257, no. 1, 1 juillet 2008 (2008-07-01), pages 55-63, XP022710515, ISSN: 0021-9517, DOI: 10.1016/J.JCAT.2008.04.006 [extrait le 2008-05-21]

EP 2 855 018 B1

**EP 2 855 018 B1**

**Description**

Domaine de l'invention

Le domaine de l'invention est celui des procédés d'hydrogénation sélective. Le procédé d'hydrogénation sélective permet de transformer les composés polyinsaturés des coupes pétrolières par conversion des composés les plus insaturés vers les alcènes correspondants en évitant la saturation totale et donc la formation des alcanes correspondants. Dans le cas d'essences de vapocraquage utilisées comme charge, l'hydrogénation sélective permet également d'hydrogéner sélectivement les alkénylaromatiques en aromatiques.

L'objet de l'invention est de proposer un catalyseur à performances améliorées, notamment en termes d'activité et de sélectivité, appliqué au procédé d'hydrogénation sélective des composés hydrocarbonés insaturés présents dans les coupes d'hydrocarbures, de préférence des coupes issues du vapocraquage. L'invention concerne plus particulièrement un procédé de préparation dudit catalyseur.

Art Antérieur

Les catalyseurs d'hydrogénation sélective sont généralement à base de métaux du groupe VIII du tableau périodique, de préférence le palladium ou le nickel. Le métal se présente sous la forme de particules métalliques déposées sur un support qui peut être un oxyde réfractaire sous forme de billes, d'extrudés ou sous des formes présentant d'autres géométries. La teneur en métal, la taille des particules de métal et la répartition de la phase active dans le support font partie des critères qui ont une importance sur l'activité et la sélectivité des catalyseurs.

Ces catalyseurs sont utilisés dans des procédés d'hydrogénation sélective par mise en contact d'une charge sur ces catalyseurs, ladite charge étant généralement des coupes C3, des coupes C4, des coupes C5 de vapocraquage et les essences de vapocraquage appelée aussi essences de pyrolyse. L'hydrogénation sélective a comme but d'hydrogéner sélectivement les composés polyinsaturés, notamment les composés acétyléniques et dioléfiniques, en alcènes correspondants. Dans le cas d'essences de vapocraquage utilisées comme charge, l'hydrogénation sélective permet également d'hydrogéner sélectivement les alkénylaromatiques en aromatiques.

Comme tout catalyseur, les catalyseurs d'hydrogénation sélective se montrent fragiles envers certains contaminants présents dans les coupes d'hydrocarbures, notamment dans les charges issues du vapocraqueur.

Le silicium est un des contaminants des charges issues du vapocraqueur. Bien qu'il soit présent en très faible concentration dans les charges, il empoisonne les catalyseurs utilisés pour les hydrogénations sélectives de ces charges. En particulier, sur les catalyseurs à base de Pd ou de Ni utilisés pour l'hydrogénation sélective des essences de vapocraquage, on peut retrouver des teneurs en silicium de 5000 ppm voire même 1.5 % pds, sur les catalyseurs "usés", déchargés des unités industrielles.

De nombreux travaux font état de l'empoissonnement de catalyseurs par le silicium. Le document "Reactions of Organosilicon Compounds on Metals : III. Selective Poisoning by Et3SiH of Catalytic Hydrogenation and Dehydrogenation" (A. Molnar, I. Bucsi, M. Bartok, F. Notheisz, G.V. Smith, Journal of Catalysis 98, 386, (1986)) décrit par exemple l'effet d'empoisonnement de catalyseurs à base de métaux du groupe VIII par le Si qui se traduit par une baisse d'activité de ces catalyseurs. Le document "Modified Activities and Selectivities of Silated-oxidized-Reduced Pd and Pt catalysts" (G.V. Smith, S. Tjandra, M. Musoiu, T. Wiltowski, F. Notheisz, M. Bartók, I. Hannus, D. Ostgard, V. Malhotra, Journal of Catalysis 161, 441 (1996)) décrit ce même phénomène et indique que les catalyseurs empoissonnés par le silicium peuvent être réactivés par un traitement d'oxydation-réduction. Le composé silicé utilisé est le triethylsilane $Et_3SiH$ ou le silane $SiH_4$, introduit de façon discontinue (pulses) sur le catalyseur sous atmosphère d'hydrogène ou inerte à haute température (250°C).

Cependant, dans certains cas, la présence de composés silicés dans les catalyseurs d'hydrogénation sélective permet d'observer un effet bénéfique pour la réaction. En effet, le document "Properties of Si-modified Pd catalyst for selective hydrogenation of acetylene" (E.W. Shin, C.H. Choi, K.S. Chang, Y.H. Na, S.H. Moon, Catalysis Today 44, 137 (1998)) décrit une augmentation de la sélectivité en éthylène dans une hydrogénation sélective de l'acétylène en éthylène par un catalyseur supporté à base de Pd, modifié par addition d'un composé de silicium. L'addition de silicium se fait par un dépôt chimique en phase vapeur (ou CVD pour "Chemical Vapour Deposition" selon le terme anglosaxon) en introduisant des quantités de $SiH_4$ de façon discontinue dans une atmosphère d'hydrogène à 250 °C, suivi d'une oxydation. Le document "Performance of Si-modified Pd catalyst in acetylene hydrogenation: catalyst deactivation behavior" (W.J. Kim, E. W. Shin, J. H. Kang, S. H. Moon, Applied Catalysis A: General 251, 305 (2003)) décrit la même réaction et divulgue une baisse de la désactivation du catalyseur Pd en cas de présence de silicium. Le document US2006/0229478 décrit un catalyseur supporté à base de Pd et de La, modifié par un composé silicé tel que le $SiH_4$, le $SiHEt_3$, ou le phénylsilane, introduit par la méthode CVD. Ce catalyseur montre une sélectivité en éthylène améliorée dans une hydrogénation sélective de l'acétylène en éthylène.

On connait par ailleurs des catalyseurs supportés à base de métaux du groupe VIII dopés par le silicium trouvant une application dans des réactions d'hydrotraitement (décrit par exemple dans les documents WO95/11753 et EP955089). Lors de la préparation de ces catalyseurs, le silicium est introduit en phase liquide par imprégnation d'un composé silicé soit sans solvant, soit en présence d'un solvant aqueux et/ou alcoolique. Dans tous les cas, les catalyseurs ainsi silicés sont par la suite soumis à une étape de calcination avant leur utilisation dans l'hydrotraitement. La calcination est effectuée sous atmosphère oxydante entre 150 et 600°C. Ils subissent ainsi une oxydation.

Le document US2012/0071700 décrit des catalyseurs supportés pour l'hydrogénation d'hydrocarbures insaturés comprenant au moins un composant métallique actif choisi parmi le palladium, le platine, le nickel, le cuivre, et le ruthénium, et au moins un composé silicé. Le composé silicé peut être introduit en phase liquide ou en phase gazeuse. Il est choisi parmi les silanes organiques, les siloxanes organiques, les silazanes organiques et les chlorosilanes organiques.

De manière surprenante, la demanderesse a découvert que des catalyseurs d'hydrogénation sélective obtenus par un procédé de préparation à l'aide de certaines molécules silicées et sous certaines conditions, notamment en phase liquide et en présence d'un solvant apolaire, permettaient d'améliorer de façon importante l'activité de ces catalyseurs dans des réactions d'hydrogénation sélective. La présente invention décrit ainsi un nouveau type de catalyseur qui, de par son procédé spécifique de préparation va permettre d'obtenir un catalyseur plus actif en hydrogénation sélective tout en gardant des sélectivités élevées. De plus, pour certains composés silicés on observe en même temps une augmentation de la sélectivité.

La mise en contact en phase liquide et en présence d'un solvant apolaire présente l'avantage d'être adaptée à des composés de silices peu volatiles. De plus, on obtient un meilleur contrôle de la quantité de composé de slice déposée par rapport à la mise en contact en phase gazeuse.

Description détaillée de l'invention

Plus particulièrement, l'invention porte sur un procédé de préparation d'un catalyseur d'hydrogénation sélective dans lequel on fournit un précurseur de catalyseur comprenant au moins un métal du groupe VIII sous forme métallique et au moins un support formé d'au moins un oxyde, ledit métal du groupe VIII étant le palladium et ledit support étant une alumine, caractérisé par le fait qu'on effectue une étape de mise en contact dudit précurseur de catalyseur sous forme métallique en phase liquide et en présence d'une atmosphère réductrice et/ou inerte, avec un solvant apolaire contenant un composé silicé, ledit composé silicé est choisi parmi

- les silanes contenant au moins une liaison Si-H et au moins une liaison Si-C répondant à la formule $Si_xH_yR_z$, dans laquelle x est un nombre entier compris entre 1 et 6, y est un nombre entier compris entre 1 et 2x+1 et z est un nombre entier compris entre 1 et 2x+1, la somme de y+z étant de 2x+2, le radical R est un radical hydrocarboné monovalent, identique ou différent pour chaque valence, et peut être choisi parmi un radical aliphatique saturé, un radical aliphatique non saturé, un radical cycloalkyle, un radical aryle et un radical arylalkyle,
- les silanols répondant à la formule $Si_x(OH)R_{2x+1}$, dans laquelle x est un nombre entier compris entre 1 et 6 et R est un radical tel que défini ci-dessus et - les siloxanes cycliques dans lequel la chaîne principale -Si-O-Si-O- forme un cycle et est composée d'unité $(R_2SiO)_n$, avec n étant un nombre entier compris entre 3 à 11, et R est un radical tel que défini ci-dessus.

Le catalyseur ainsi préparé ne nécessite pas d'étape de calcination par la suite. On entend ici par calcination un traitement thermique sous atmosphère oxydante à une température supérieure à 150°C.

Ledit métal du groupe VIII se présente sous la forme de particules métalliques déposées sur ledit support. De manière générale, la teneur en métal du groupe VIII dans le catalyseur est comprise entre 0,01 et 50% poids de la masse du catalyseur préparé selon le procédé de l'invention, de manière préférée entre 0,05 et 30% poids de la masse du catalyseur.

De manière préférée, la teneur en palladium dans ledit catalyseur préparé selon le procédé l'invention est avantageusement comprise entre 0,01 et 5 % poids de la masse du catalyseur, de préférence entre 0,05 et 2 % poids de la masse du catalyseur, et de manière encore plus préférée entre 0,05 et 1 % poids de la masse du catalyseur.

La phase active dudit catalyseur peut comprendre en outre au moins un métal additionnel choisi parmi les métaux du groupe VIII, les métaux du groupe IB et/ou de l'étain. De manière préférée, le métal additionnel du groupe VIII est choisi parmi le platine, le ruthénium et le rhodium, et le nickel. Avantageusement, le métal additionnel du groupe IB est choisi parmi le cuivre, l'or et l'argent. Le(s)dit(s) métal(ux) additionnel(s) du groupe VIII et/ou du groupe IB est(sont) préférentiellement présent(s) dans une teneur représentant de 0,01 à 20 % poids de la masse du catalyseur, de préférence de 0,05 à 10 % poids de la masse du catalyseur et de manière encore plus préférée de 0,05 à 5 % poids de la masse dudit catalyseur. L'étain est préférentiellement présent dans une teneur représentant de 0,02 à 15 % poids de la masse du catalyseur, de telle sorte que le ratio Sn/métal(ux) du groupe VIII soit compris entre 0,01 et 0,2, de préférence entre 0,025 à 0,055, et de manière encore plus préférée entre 0,03 à 0,05.

Il est préférable de déposer le palladium dans une fine croûte à la périphérie des grains de support, tel que par exemple décrit dans le document FR2922784. La teneur en palladium dans ce catalyseur est comprise entre 0,05 et 2 % poids et au moins 80 % poids de palladium est réparti dans une croûte à la périphérie du support. L'épaisseur de cette croûte est généralement comprise entre 10 et 1000 $\mu$m, de préférence entre 20 et 600 $\mu$m, et de manière encore plus préférée entre 20 et 200 $\mu$m.

Un catalyseur particulièrement préféré est décrit dans le document FR2922784 et comprend du palladium en fine croûte et sous sa forme métallique, ledit catalyseur comprend au moins un métal sélectionné dans le groupe constitué par les alcalins et les alcalino-terreux, un support poreux comprenant au moins un oxyde réfractaire sélectionné dans le groupe constitué par la silice, l'alumine et la silice-alumine, dans lequel la surface spécifique du support poreux est comprise entre 50 et 210 m$^2$/g, la teneur en palladium dans le catalyseur est comprise entre 0,05 et 2 % poids, au moins 80 % poids de Palladium est réparti dans une croûte à la périphérie du support, l'épaisseur de ladite croûte est comprise entre 20 et 200 $\mu$m, la dispersion métallique D est comprise entre 25% et 70%, la densité de particules de palladium dans la croûte est comprise entre 1500 et 4100 particules de palladium par $\mu$m$^2$, la somme des teneurs en métaux alcalins et/ou alcalino-terreux dans le catalyseur est comprise entre 0,05 et 5 % poids et ledit métal alcalin et/ou alcalino-terreux est réparti de manière homogène à travers le support avec un coefficient R compris entre 0,8 et 1,2, ledit coefficient R étant défini dans FR2922784.

Selon l'invention, ledit support est une alumine.

Le volume poreux du support est généralement compris entre 0,1 cm$^3$/g et 1,5 cm$^3$/g, de préférence compris entre 0,5 cm$^3$/g et 1,3 cm$^3$/g.

La surface spécifique du support est généralement comprise entre 10 m$^2$/g et 250 m$^2$/g, de préférence entre 30 m$^2$/g et 220 m$^2$/g.

Ledit support poreux se présente avantageusement sous forme de billes, d'extrudés, de pastilles, ou d'agglomérats irréguliers et non sphériques dont la forme spécifique peut résulter d'une étape de concassage. De manière très avantageuse, ledit support se présente sous forme de billes ou d'extrudés.

Selon l'invention, l'étape d'introduction du silicium dans le précurseur de catalyseur est effectuée en présence d'une atmosphère réductrice et/ou inerte et par la mise en contact en phase liquide du précurseur de catalyseur supporté comprenant au moins un métal du groupe VIII avec un solvant apolaire contenant un composé silicé, ledit composé silicé est choisi parmi les silanes contenant au moins une liaison Si-H et au moins une liaison Si-C, les silanols et les siloxanes cycliques tels que décrits ci-après.

Selon une première variante, le composé silicé est choisi parmi les silanes contenant au moins une liaison Si-H et au moins une liaison Si-C répondant à la formule Si$_x$H$_y$R$_z$, dans laquelle x est un nombre entier compris entre 1 et 6, y est un nombre entier compris entre 1 et 2x+1 et z est un nombre entier compris entre 1 et 2x+1, la somme de y+z étant de 2x+2. Le radical R est un radical hydrocarboné monovalent, identique ou différent pour chaque valence, et peut être choisi parmi un radical aliphatique saturé, un radical aliphatique non saturé, un radical cycloalkyle, un radical aryle et un radical arylalkyle. De préférence, les silanes répondent à la formule Si$_x$H$_y$R$_z$, dans laquelle x est un nombre entier compris entre 1 et 3, de préférence x est égal à 1.

Par "radical aliphatique saturé", on entend pour R une chaîne hydrocarbonée linéaire ou ramifiée ayant de 1 à 15 atomes de carbone, de préférence de 1 à 10 atomes de carbone et encore plus préférentiellement de 1 à 4 atomes de carbone. Les radicaux aliphatiques saturés sont de préférence choisis parmi les groupes méthyle, éthyle, propyle comprenant le n-propyle et l'isopropyle, butyle comprenant le n-butyle, l'isobutyle, le sec-butyle et le ter-butyle, et de façon très préférée parmi les groupes méthyle et éthyle.

Par "radical aliphatique non saturé", on entend pour R une chaîne hydrocarbonée linéaire ou ramifiée ayant de 1 à 15 atomes de carbone, de préférence de 1 à 10 atomes de carbone et encore plus préférentiellement de 1 à 4 atomes de carbone. Des radicaux aliphatiques non saturés préférés sont avantageusement choisis parmi les groupes vinyle, allyle, méthallyle.

Par « radical cycloalkyle», on entend pour R un groupe hydrocarboné cyclique saturé, monocyclique ayant de 3 à 10 atomes de carbone de préférence, notamment un groupe cyclopentyle ou cyclohexyle.

Par « radical aryle », on entend pour R un groupe aromatique ayant de 6 à 20 atomes de carbone, de préférence mono- ou bicyclique, et de préférence phényle ou naphtyle.

Par « radical arylalkyle », on entend pour R un groupe hydrocarboné, linéaire ou ramifié porteur d'un cycle aromatique monocyclique, ayant de 7 à 12 atomes de carbone, la chaîne aliphatique comprenant 1 à 6 atomes de carbone. Un groupe arylalkyle préféré est le groupe benzyle, tolyle, xylyle, éthylphényle.

Plus particulièrement, le composé silicé peut être choisi parmi le triméthylsilane, le triéthylsilane, le tripropylsilane, le tributylsilane, le méthyldiéthylsilane, le méthyldipropylsilane, le méthyldibutylsilane, le diméthyléthylsilane, le diméthylpropylsilane, le diméthylbutylsilane, l'éthyldipropylsilane, l'éthyldibutylsilane, le diéthylpropylsilane, le diéthylbutylsilane, le propyldibutylsilane, le dipropylbutylsilane, le diméthylphénylsilane, le diméthylcyclohexanesilane, le diéthylphénylsilane, le méthyléthylphénylsilane, le méthyldiphénylsilane, l'éthyldiphénylsilane, le méthylsilane, l'éthylsilane, le propylsilane, le butylsilane, le diméthylsilane, le diéthylsilane, le dipropylsilane, le dibutylsilane, le méthyléthylsilane, le méthylpropylsilane, le méthylbutylsilane, l'éthylpropylsilane, l'éthylbutylsilane, le propylbutylsilane, le méthyldisilane, le diméthyldisilane, le triméthyldisilane, le tétraméthyldisilane, le pentaméthyldisilane, l'éthyldisilane, le diéthyldisilane, le triéthyldisilane, le tétraéthyldisilane, le pentaéthyldisilane, le propyldisilane, le dipropyldisilane, le tripropyldisilane, le tétrapropyldisilane, le pentapropyldisilane, le butyldisilane, le dibutyldisilane, le tributyldisilane, le tétrabutyldisilane, le pentabutyldisilane, le méthyltrisilane, le diméthyltrisilane, le triméthyltrisilane, le tétraméthyltrisilane, le pentaméthyltrisilane, le hexaméthyltrisilane, le heptaméthyltrisilane, l'éthyltrisilane, le diéthyltrisilane, le triéthyltrisilane, le tétraéthyltrisilane, le pentaéthyltrisilane, l'hexaéthyltrisilane ou encore l'heptaéthyltrisilane. On préfère particulièrement le triéthylsilane.

Selon une autre variante, le composé silicé est choisi parmi les silanols répondant à la formule $Si_x(OH)R_{2x+1}$, dans laquelle x est un nombre entier compris entre 1 et 6, et R est un radical tel que défini ci-dessus. De préférence, les silanes répondent à la formule $Si_x(OH)R_{2x+1}$, dans laquelle x est un nombre entier compris entre 1 et 3, de préférence x est égal à 1. De préférence, R est un groupe choisi parmi les groupes méthyle, éthyle, propyle comprenant le n-propyle et l'isopropyle, butyle comprenant le n-butyle, l'isobutyle, le sec-butyle et le ter-butyle, et de façon très préférée parmi les groupes méthyle et éthyle.

Plus particulièrement, le composé silicé peut être choisi parmi le triméthylsilanol, le triéthylsilanol, le tripropylsilanol, le tributylsilanol, le méthyldiéthylsilanol, le méthyldipropylsilanol, le méthyldibutylsilanol, le diméthyléthylsilanol, le diméthylpropylsilanol, le diméthylbutylsilanol, l'éthyldipropylsilanol, l'éthyldibutylsilanol, le diéthylpropylsilanol, le diéthylbutylsilanol, le propyldibutylsilanol, le dipropylbutylsilanol, le diméthylphénylsilanol, le diméthylcyclohexanesilanol, le diéthylphénylsilanol, le méthyléthylphénylsilanol, le méthyldiphénylsilanol, l'éthyldiphénylsilanol. On préfère particulièrement le triéthylsilanol.

Selon une autre variante, le composé silicé est choisi parmi les siloxanes cycliques dans lequel la chaîne principale -Si-O-Si-O- forme un cycle et est composée de préférence d'unité $(R_2SiO)_n$, avec n étant un nombre entier compris entre 3 à 11, de préférence entre 3 et 8, et R est un radical tel que défini ci-dessus. De préférence, R est un groupe méthyle.

Plus particulièrement, le composé silicé peut être choisi parmi l'hexaméthylcyclotrisiloxane, l'octaméthylcyclotetrasiloxane, le décaméthylcyclopentasiloxane, le dodécaméthylcyclohexasiloxane, le tetradécaméthylcycloheptasiloxane et l'hexadécaméthylcyclooctasiloxane. On préfère particulièrement l'octaméthylcyclotetrasiloxane.

Le solvant apolaire est préférentiellement choisi parmi un solvant hydrocarboné, par exemple un solvant hydrocarboné aliphatique tel que l'hexane, l'heptane, l'octane, le nonane, le décane, un solvant hydrocarboné cyclique tel que le cyclohexane, un solvant hydrocarboné aromatique tel que le benzène, le toluène ou le xylène, une essence partiellement saturée, un effluent partiellement hydrogéné issu d'un procédé d'hydrogénation sélective effectué par la suite, ou encore un mélange de ces solvants. De préférence, on utilise l'heptane, une essence partiellement saturée ou encore l'effluent partiellement hydrogéné issu du procédé d'hydrogénation sélective effectué par la suite.

De préférence, le composé silicé est présent dans le solvant apolaire dans une concentration entre 0,01 et 10 % pds de silicium, de préférence 0,1 et 10 % pds de silicium, de préférence entre 0,5 et 8 % pds de silicium, et de manière encore plus préférée entre 1 et 5 % pds de silicium.

Préférentiellement, l'étape de mise en contact est effectuée à une température comprise entre 20 et 200°C, de préférence entre 20°C et 180°C, et de manière encore plus préférée entre 50 et 180°C. La pression est généralement comprise entre la pression atmosphérique et la pression à laquelle le procédé d'hydrogénation sélective sera opéré, c'est-à dire, comprise entre 0,3 et 6,5 MPa.

Le temps de contact du précurseur de catalyseur supporté avec le solvant polaire contenant le composé silicé est avantageusement supérieur à 15 minutes, de préférence supérieur à 1 heure, si la réaction est effectuée en système discontinu (batch); en général il est de 5 heures. Dans le cas d'une utilisation en réacteur fixe dans des conditions d'un système continu, la vitesse spatiale horaire (VVH) étant définie comme volume de charge/volume de catalyseur/heure, est avantageusement comprise entre 0,1 et 10 $h^{-1}$, de préférence entre 0,2 et 5 $h^{-1}$, et de manière encore plus préférée entre 1 et 5 $h^{-1}$.

Le précurseur de catalyseur est mis en contact avec le solvant polaire contenant le composé silicé en présence d'une atmosphère réductrice (par exemple en présence d'hydrogène, pur ou dilué) et/ou en présence d'une atmosphère inerte (par exemple en présence d'azote).

Les catalyseurs ainsi préparés ne nécessitent pas d'étape de calcination par la suite. On entend ici par calcination un traitement thermique sous atmosphère oxydante à une température supérieure à 150°C.

Le catalyseur obtenu à l'issue du procédé de préparation de l'invention est avantageusement utilisé directement à l'issue de ladite étape d'introduction du silicium dans une unité réactionnelle réalisant la conversion d'une charge hydrocarbonée, en particulier dans une unité réactionnelle réalisant l'hydrogénation sélective d'une charge d'hydrocarbures polyinsaturés. Ledit catalyseur préparé selon le procédé de l'invention peut aussi être stocké à l'air puis réduit avant utilisation. La réduction est alors effectuée sous courant de gaz réducteur, de préférence sous hydrogène, pur ou dilué, à haute température, typiquement supérieure ou égale à 50°C pendant une durée supérieure ou égale à 2 heures.

Le procédé d'introduction de silicium selon l'invention est effectué sur un précurseur de catalyseur supporté présent sous sa forme métallique (ou réduite). On entend par forme métallique que les métaux présents sur le support sont au moins partiellement, et de préférence majoritairement, dans un degré d'oxydation de zéro.

L'étape d'introduction du silicium peut être précédée par des étapes classiques de préparation de catalyseurs d'hydrogénation sélective, connues par l'homme du métier.

Ainsi, selon une variante préférée, le procédé de préparation du catalyseur comprend en outre les étapes suivantes, lesdites étapes étant effectuées avant l'étape de mise en contact :

a) au moins une étape, dans laquelle on imprègne une solution contenant au moins un précurseur dudit métal du groupe VIII, sur ledit support,

b) au moins une étape dans laquelle on sèche le support imprégné issu de l'étape a),

c) au moins une étape, dans laquelle on calcine le support séché issu de l'étape b) de manière à obtenir au moins ledit métal dudit groupe VIII sous forme oxyde,

d) au moins une étape, dans laquelle on effectue un traitement réducteur du support calciné issu de l'étape c) par mise en contact avec un gaz réducteur de manière à obtenir au moins ledit métal dudit groupe VIII sous forme métallique.

Le procédé de préparation peut comprendre en outre une étape e) dans laquelle on effectue une passivation du précurseur de catalyseur par un composé soufré. Cette étape de passivation peut être effectuée soit après l'étape c) de calcination et avant l'étape d) de traitement réducteur du précurseur de catalyseur, soit après l'étape d) de traitement réducteur du précurseur de catalyseur, ou encore après l'étape d'introduction du silicium.

Les différentes étapes de préparation a), b), c), d), et e) du précurseur de catalyseur supporté sous forme métallique sont décrites par la suite.

Étape d'imprégnation

L'étape a) d'imprégnation peut être réalisée par toute méthode bien connue de l'homme du métier. En particulier l'étape a) peut être réalisée par imprégnation en excès ou par imprégnation à sec. L'imprégnation peut être réalisée en une ou plusieurs imprégnations successives.

De manière préférée, ladite étape a) est réalisée par imprégnation à sec, laquelle consiste à mettre en contact le support avec la solution aqueuse ou organique (tel qu'un solvant alcoolique) avec un volume égal au volume poreux du support à imprégner.

Lorsqu'il est introduit en solution organique, ledit précurseur du métal du groupe VIII, est par exemple l'oxalate ou l'acétate dudit métal du groupe VIII. De manière préférée, ledit précurseur du métal du groupe VIII est introduit en solution aqueuse, par exemple sous forme de nitrate, de carbonate, d'acétate, de chlorure, d'oxalate, d'hydroxyde ou de tout autre dérivé inorganique soluble en solution aqueuse.

Dans le cas où ledit métal du groupe VIII est le palladium, on utilise avantageusement comme précurseur de palladium en phase aqueuse un précurseur choisi parmi le nitrate de palladium, le chlorure de palladium, le sulfate de palladium, de manière préférée le nitrate de palladium.

Dans le cas où ledit métal du groupe VIII est le nickel, on utilise avantageusement comme précurseur de nickel en phase aqueuse un précurseur choisi parmi le nitrate de nickel, le chlorure de nickel, le carbonate de nickel, l'acétate de nickel et l'hydroxyde de nickel, de manière préférée le nitrate de nickel.

Lorsqu'on souhaite obtenir un catalyseur à base de palladium en croûte, on utilise par exemple la méthode d'imprégnation colloïdale telle que décrite dans FR2922784 et selon laquelle on prépare une suspension colloïdale d'oxyde de palladium ou d'hydroxyde de palladium en phase aqueuse par mélange d'une solution aqueuse 1 comprenant au moins un hydroxyde sélectionné dans le groupe constitué par les hydroxydes d'alcalins, de préférence de sodium, et les hydroxydes d'alcalino-terreux et d'une solution aqueuse 2 comprenant au moins un sel précurseur du palladium, la solution 2 puis la solution 1 étant versées dans un appareillage ou les solutions 1 et 2 étant versées simultanément dans un appareillage, ladite suspension colloïdale étant ensuite utilisée pour imprégner le support.

Étape de séchage

L'étape b) de séchage du support imprégné issu de l'étape a) est réalisée préférentiellement selon le procédé de préparation de l'invention à une température comprise entre 20 et 160°C, de préférence entre 20 et 130°C. L'étape b) de séchage est préférentiellement réalisée pendant une durée comprise entre 1 et 24 heures, de préférence entre 1 et 20 heures. Le séchage est effectué sous air ou sous atmosphère inerte (azote par exemple).

Etape de calcination

A l'issue de l'étape b) de séchage, une étape c) de calcination du support est réalisée à une température comprise entre 150 et 800°C, de préférence entre 250 et 600°C et de manière très préférée entre 300 et 600°C. Généralement, la calcination se fait sous air. Elle est préférentiellement réalisée pendant une durée comprise entre 1 et 6 heures. Le précurseur de catalyseur obtenu à l'issue de ladite étape c) se trouve à l'état oxyde.

Etape de réduction

Préalablement à l'introduction du silicium dans le précurseur de catalyseur et de son utilisation subséquente dans le réacteur catalytique et la mise en oeuvre du procédé d'hydrogénation sélective, le précurseur de catalyseur est soumis au moins à une étape d) de traitement réducteur, par mise en contact avec un gaz réducteur, par exemple avec de l'hydrogène, pur ou dilué, à haute température, typiquement supérieure ou égale à 50°C pendant une durée supérieure ou égale à 2 heures. Ce traitement permet d'activer ledit précurseur et de former des particules de métal, en particulier de métal du groupe VIII, à l'état zéro valent. Ledit traitement réducteur peut être réalisé *in-situ* ou *ex-situ* c'est-à-dire avant le chargement du catalyseur dans le réacteur d'hydrogénation sélective. Ladite étape d) de traitement réducteur peut être mise en oeuvre sur le précurseur de catalyseur ayant été soumis ou non à l'étape e) de passivation.

Étape de passivation

Le procédé de préparation selon l'invention peut comprendre une étape e) de passivation du précurseur de catalyseur par un composé soufré, qui peut être effectuée soit après l'étape c) de calcination et avant l'étape d) de traitement réducteur du précurseur de catalyseur, soit après l'étape d) de traitement réducteur du précurseur de catalyseur, soit encore après l'étape de mise en contact. L'étape de passivation au soufre permet d'améliorer la sélectivité des catalyseurs.

Lorsque l'étape e) de passivation est effectuée après l'étape c) de calcination et avant l'étape d) de traitement réducteur du précurseur de catalyseur, l'étape e) est de préférence effectuée *ex-situ,* c'est-à-dire avant chargement du catalyseur dans l'unité réactionnelle d'hydrogénation sélective. Ladite étape e) est réalisée par la mise en oeuvre de méthodes connues de l'homme du métier et notamment, à titre d'exemple par la mise en oeuvre de l'une des méthodes décrites dans les documents EP0466567, US5153163, FR2676184, WO2004/098774 ou encore dans le document EP0707890. De manière préférée, ladite étape e) est effectuée par la mise en contact du précurseur de catalyseur, obtenu à l'issue de la mise en oeuvre de l'étape c) avec au moins une solution comprenant au moins un réducteur organique et au moins un composé soufré. De manière très préférée, ladite étape e) est réalisée par imprégnation du précurseur de catalyseur, obtenu à l'issue de la mise en oeuvre de l'étape c) avec ladite solution. Le réducteur organique présent dans ladite solution est par exemple choisi parmi l'acide formique, le formaldéhyde, l'acide acétique, l'éthanol, le méthanol, le formiate d'éthyl et le formiate de méthyle. Le composé soufré présent dans ladite solution est par exemple un composé de formule HO-R1-S-S-R2-OH (où R1 et R2 peuvent être tout type de radical organique) tel que le di-éthanol-di-sulfure (DEODS) ou encore un composé organique polysulfure de formule R-S(n)-R' où R et R' sont des radicaux organiques et n est compris entre 3 et 20, par exemple le dodecyl polysulfure. La quantité de soufre introduite est telle que le catalyseur passivé par le soufre comprenne de 0,2 à 2% poids de soufre. La quantité de réducteur organique introduite est telle que le catalyseur passivé comprenne entre 100 ppm (partie par million) et 50% poids dudit réducteur. Après introduction dudit composé soufré sur le catalyseur, ledit précurseur de catalyseur est ensuite soumis à un traitement thermique réalisé à une température comprise entre 100 et 200°C pendant une durée comprise entre 30 minutes et 3 heures. Lorsque l'étape e) de passivation est effectuée après l'étape d) de traitement réducteur du précurseur de catalyseur, ou encore après l'étape d'introduction du silicium, l'étape e) est de préférence effectuée *in-situ,* c'est-à-dire au sein du même réacteur que celui où est opérée la réaction d'hydrogénation sélective. Ladite étape de passivation est effectuée par injection d'au moins un composé soufré avant ou après la mise en contact dudit précurseur de catalyseur avec le solvant apolaire contenant le composé silicé. Le composé soufré est par exemple choisi parmi les composés suivants : thiophène, thiophane, alkylmonosulfures tels que diméthylsulfure, diéthylsulfure, dipropylsulfure et propylméthylsulfure.

Procédé d'hydrogénation sélective

La présente invention concerne également un procédé d'hydrogénation sélective d'une charge d'hydrocarbures polyinsaturés contenant au moins 3 atomes de carbone par molécule et ayant un point d'ébullition final inférieur ou égal à 250°C, par mise en contact de ladite charge avec au moins le catalyseur préparé selon le procédé de préparation de l'invention.

La charge d'hydrocarbures polyinsaturés, traitée dans le procédé d'hydrogénation sélective, est préférentiellement choisie parmi la coupe C3 de vapocraquage, la coupe C4 de vapocraquage, la coupe C5 de vapocraquage et les essences de vapocraquage encore appelées essences de pyrolyse. L'ensemble de ces coupes et les essences de vapocraquage contiennent au moins 3 atomes de carbone par molécule et présentent un point d'ébullition final inférieur ou égal à 250°C. Plus précisément, lesdits hydrocarbures polyinsaturés présents dans ladite charge traitée sont en particulier des composés comportant au moins une fonction acétylénique (c'est-à-dire au moins une triple liaison) et/ou au moins une fonction diénique (c'est-à-dire au moins deux double liaisons). En particulier, ladite charge d'hydrocarbures polyinsaturés peut comprendre au moins un type de composé contenant à la fois une fonction acétylène et une fonction diénique par molécule. La charge essences de pyrolyse peut en plus comprendre des alkénylaromatiques.

Les procédés de conversion des hydrocarbures tels que le procédé de vapocraquage, dont est préférentiellement issue ladite charge hydrocarbonée à traiter selon le procédé d'hydrogénation sélective utilisant le catalyseur préparé selon le procédé de l'invention, sont opérés à haute température et produisent une grande variété de molécules mono-insaturées telles que le propylène, les butènes linéaires, l'isobutène, les pentènes ainsi que des molécules mono-insaturées contenant jusqu'à environ 15 atomes de carbone. En parallèle sont également formés des composés polyinsaturés ayant plusieurs doubles liaisons et/ou au moins une triple liaison, en particulier de l'acétylène, du propadiène et du méthylacétylène (ou propyne), du 1,2-butadiène et 1,3-butadiène, du butyne, du vinylacétylène et de l'éthylacétylène, du pentadiène ainsi que d'autres composés polyinsaturés présents dans les essences de vapocraquage, en particulier des composés styréniques et indéniques.

Tous ces composés polyinsaturés doivent être éliminés pour permettre l'utilisation de ces différentes coupes dans les procédés de pétrochimie tels que les unités de polymérisation, ou dans les procédés de raffinage.

La coupe C3 de vapocraquage, avantageusement utilisée pour la mise en oeuvre du procédé d'hydrogénation sélective selon l'invention, présente par exemple la composition moyenne suivante : de l'ordre de 90% poids de propylène, de l'ordre de 3 à 8% poids de propadiène et de méthylacétylène, le reste étant essentiellement du propane. Dans certaines coupes C3, entre 0,1 et 2% poids de composés en C2 et de composés en C4 peut aussi être présent.

La coupe C4 de vapocraquage, avantageusement utilisée pour la mise en oeuvre du procédé d'hydrogénation sélective selon l'invention, présente par exemple la composition massique moyenne suivante : 1 % poids de butane, 46,5% poids de butène, 51 % poids de butadiène, 1,3% poids de vinylacetylène (VAC) et 0,2% poids de butyne. Dans certaines coupes C4, entre 0,1 et 2% poids de composés en C3 et de composés en C5 peut aussi être présent.

La coupe C5 de vapocraquage, avantageusement utilisée pour la mise en oeuvre du procédé d'hydrogénation sélective selon l'invention, présente par exemple la composition suivante : 21% poids de pentanes, 45% poids de pentènes, 34% poids de pentadiènes.

L'essence de vapocraquage ou essence de pyrolyse, avantageusement utilisée pour la mise en oeuvre du procédé d'hydrogénation sélective selon l'invention, correspond à une coupe hydrocarbonée dont la température d'ébullition est généralement comprise entre 0°C et 250°C, de préférence entre 10°C et 220°C. Les hydrocarbures polyinsaturés présents dans ladite essence de vapocraquage sont en particulier des composés dioléfiniques (butadiène, isoprène, cyclopentadiène...), des composés styréniques (styrène, alpha-méthylstyrène...) et des composés indéniques (indène...). L'essence de vapocraquage comprend généralement la coupe C5-C12 avec des traces de C3, C4, C13, C14, C15 (par exemple entre 0,1 et 3% poids pour chacune de ces coupes). Par exemple, l'essence de vapocraquage peut avantageusement présenter la répartition suivante, selon les fonctions chimiques présentes dans les composés de la charge hydrocarbonée (en % poids) :

- Paraffines + naphtènes : 10-25
- Aromatiques : 50-70
- Mono-oléfines : 5-20
- Dioléfines : 10-25
- Alkénylaromatiques : 2-10
- Soufre 5 - 500 ppm.

Le procédé d'hydrogénation sélective selon l'invention vise à éliminer lesdits hydrocarbures polyinsaturés présents dans ladite charge à hydrogéner en procédant à la conversion desdits hydrocarbures polyinsaturés vers les alcènes correspondants en évitant la saturation totale desdits hydrocarbures de manière à éviter la formation des alcanes correspondants.

Par exemple, lorsque ladite charge est une coupe C3, le procédé d'hydrogénation sélective selon l'invention vise à hydrogéner sélectivement le propadiène et le méthylacétylène. Dans le cas d'une coupe C4, on vise à éliminer le butadiène, le vinylacétylène (VAC) et le butyne, dans le cas d'une coupe C5, on vise à éliminer les pentadiènes. Lorsque ladite charge est une essence de vapocraquage, le procédé d'hydrogénation sélective selon l'invention vise à hydrogéner sélectivement lesdits hydrocarbures polyinsaturés présents dans ladite charge à traiter de manière à ce que les composés dioléfiniques soient partiellement hydrogénés en mono-oléfines et que les composés styréniques et indéniques soient partiellement hydrogénés en composés aromatiques correspondants.

La mise en oeuvre technologique du procédé d'hydrogénation sélective selon l'invention est par exemple réalisée par injection, en courant ascendant ou descendant, de la charge d'hydrocarbures polyinsaturés et de l'hydrogène dans au moins un réacteur à lit fixe. Ledit réacteur peut être de type isotherme ou de type adiabatique. Un réacteur adiabatique est préféré. La charge d'hydrocarbures polyinsaturés peut avantageusement être diluée par une ou plusieurs ré-injection(s) de l'effluent, issu dudit réacteur où se produit la réaction d'hydrogénation sélective, en divers points du réacteur, situés entre l'entrée et la sortie du réacteur. La mise en oeuvre technologique du procédé d'hydrogénation sélective selon l'invention peut également être avantageusement réalisée par l'implantation d'au moins dudit catalyseur préparé selon le procédé de l'invention supporté dans une colonne de distillation réactive ou dans des réacteurs - échangeurs. Le flux d'hydrogène peut être introduit en même temps que la charge à hydrogéner et/ou en un point différent du réacteur.

Dans le cas où la charge est une coupe C3 de vapocraquage, une coupe C4 de vapocraquage, une coupe C5 de vapocraquage ou une essence de vapocraquage, le procédé d'hydrogénation sélective selon l'invention est mis en oeuvre en phase liquide dans les conditions opératoires suivantes : une pression totale comprise entre 0,3 MPa et 6,5 MPa, plus préférentiellement comprise entre 1 et 5 MPa, une température comprise entre 20 et 250°C et un rapport molaire hydrogène /(hydrocarbures polyinsaturés à hydrogéner) compris entre 0,1 et 4, de préférence entre 1 et 2. La vitesse spatiale horaire (définie comme le rapport du débit volumique de charge sur le volume de catalyseur par heure), établie dans ces conditions, est généralement comprise entre 0,2 et 100 h$^{-1}$. Dans le cas d'une essence de vapocraquage, la vitesse spatiale horaire est généralement comprise entre 0,5 et 20 h$^{-1}$, de préférence entre 1 et 10 h$^{-1}$ et de manière encore plus préférée entre 2 et 10 h$^{-1}$.

L'invention est illustrée par les exemples qui suivent sans pour autant en limiter la portée. Ces exemples montrent une augmentation de l'activité dans une réaction d'hydrogénation sélective pour les catalyseurs préparés selon l'invention, ainsi qu'une augmentation de la sélectivité pour certains d'entre eux.

**Exemple 1 (comparatif) : préparation d'un catalyseur de référence A**

Un catalyseur commercial de type Pd / alumine (LD265, Axens) est utilisé comme catalyseur de référence. Ledit catalyseur commercial présente une teneur en palladium égale à 0,3 %poids, une surface spécifique égale 70 m$^2$/g, un volume poreux total égal à 0,6 cm$^3$/g et un diamètre de billes compris entre 2 et 4 mm.

Ledit catalyseur commercial est broyé et tamisé pour ne conserver que la granulométrie comprise entre 200 et 355 micromètres. Une quantité de 1 g de ce catalyseur commercial est traitée sous un flux d'hydrogène avec un débit d'hydrogène de 1 NL/h, à 150 °C durant 2 heures, avec une montée en température de 300 °C/h. L'échantillon réduit ainsi obtenu est nommé catalyseur A. Il est directement introduit dans l'autoclave en vue du test d'hydrogénation décrit dans l'exemple 12, dans 140 mL d'heptane, sans aucun contact avec l'air.

**Exemple 2 (selon l'invention) : préparation d'un catalyseur B par mise en contact avec une solution de triéthylsilane dans l'heptane**

Le catalyseur A, sous forme métallique, décrit à l'exemple 1, est introduit sans aucun contact avec l'air dans l'autoclave utilisé pour le test d'hydrogénation dans 140 mL d'heptane. Une quantité de 5.7 mL de triéthylsilane (SiH(C$_2$H$_5$)$_3$, n° CAS 617-86-7) est introduite dans l'heptane, de façon à obtenir une concentration en Si égale à 1 %poids environ. L'autoclave est ensuite fermé, purgé, puis pressurisé sous 10 bar (1 MPa) d'hydrogène, et porté à la température de 50°C. Cette température est maintenue pendant 5 heures, sous agitation (1600 tour/min). Le catalyseur ainsi obtenu est nommé catalyseur B. Il est directement utilisé dans le test d'hydrogénation décrit dans l'exemple 12.

**Exemple 3 (selon l'invention) : préparation d'un catalyseur C par mise en contact avec une solution de triéthylsilane dans l'heptane**

Cette préparation est identique à celle décrite dans l'exemple 2 à l'exception de la concentration en triéthylsilane. Une quantité de 17.4 mL de triéthylsilane est introduite dans l'heptane, de façon à obtenir une concentration en Si égale à 3 %poids environ. Le catalyseur ainsi obtenu est nommé catalyseur C. Il est directement utilisé dans le test d'hydrogénation décrit dans l'exemple 12.

**Exemple 4 (selon l'invention) : préparation d'un catalyseur D par mise en contact avec une solution de triéthylsilanol dans l'heptane**

Le catalyseur A, sous forme métallique, décrit à l'exemple 1, est introduit sans aucun contact avec l'air dans l'autoclave utilisé pour le test d'hydrogénation dans 140 mL d'heptane. Une quantité de 5.3 mL de triéthylsilanol (SiOH(C$_2$H$_5$)$_3$, n° CAS 597-52-4) est introduite dans l'heptane, de façon à obtenir une concentration en Si égale à 1 %poids environ. L'autoclave est ensuite fermé, purgé, puis pressurisé sous 10 bar (1 MPa) d'hydrogène, et porté à la température de 50°C. Cette température est maintenue pendant 5 heures, sous agitation (1600 tours/min). Le catalyseur ainsi obtenu est nommé catalyseur D. Il est directement utilisé dans le test d'hydrogénation décrit dans l'exemple 12.

**Exemple 5 (selon l'invention) : préparation d'un catalyseur E par mise en contact avec une solution de triéthylsilanol dans l'heptane**

Cette préparation est identique à celle décrite dans l'exemple 4 à l'exception de la concentration en triéthylsilanol. Une quantité de 16.6 mL de triéthylsilanol est introduite dans l'heptane, de façon à obtenir une concentration en Si égale à 3 %poids environ. Le catalyseur ainsi obtenu est nommé catalyseur E. Il est directement utilisé dans le test d'hydrogénation décrit dans l'exemple 12.

**Exemple 6 (selon l'invention) : préparation d'un catalyseur F par mise en contact avec une solution de triéthylsilanol dans l'heptane**

Cette préparation est identique à celle décrite dans l'exemple 4 à l'exception de la concentration en triéthylsilanol. Une quantité de 28.3 mL de triéthylsilanol est introduite dans l'heptane, de façon à obtenir une concentration en Si égale à 5 %poids environ. Le catalyseur ainsi obtenu est nommé catalyseur F. Il est directement utilisé dans le test d'hydrogénation décrit dans l'exemple 12.

**Exemple 7 (selon l'invention) : préparation d'un catalyseur G par mise en contact** avec **une solution d'octaméthylcyclotétrasiloxane dans l'heptane**

Le catalyseur A, sous forme métallique, décrit à l'exemple 1, est introduit sans aucun contact avec l'air dans l'autoclave utilisé pour le test d'hydrogénation dans 140 mL d'heptane. Une quantité de 2.7 mL d'octaméthylcyclotétra-siloxane ($C_8H_{24}O_4Si_4$, n° CAS 556-67-2) est introduite dans l'heptane, de façon à obtenir une concentration en Si égale à 1 %poids environ. L'autoclave est ensuite fermé, purgé, puis pressurisé sous 10 bar (1 MPa) d'hydrogène, et porté à la température de 50°C. Cette température est maintenue pendant 5 heures, sous agitation (1600 tours/min). Le catalyseur ainsi obtenu est nommé catalyseur G. Il est directement utilisé dans le test d'hydrogénation décrit dans l'exemple 12.

**Exemple 8 (selon l'invention) : préparation d'un catalyseur H par mise en contact avec une solution d'octaméthylcyclotétrasiloxane dans l'heptane**

Cette préparation est identique à celle décrite dans l'exemple 7 à l'exception des conditions opératoires de la mise en contact avec la solution d'octaméthylcyclotétrasiloxane dans l'heptane. L'autoclave est pressurisé sous 10 bar (1 MPa) d'hydrogène, et porté à la température de 70°C. Cette température est maintenue pendant 7 heures, sous agitation (1000 tours/min). Le catalyseur ainsi obtenu est nommé catalyseur H. Il est directement utilisé dans le test d'hydrogénation décrit dans l'exemple 12.

**Exemple 9 (selon l'invention) : préparation d'un catalyseur I par mise en contact avec une solution de octaméthylcyclotétrasiloxane dans l'heptane**

Cette préparation est identique à celle décrite dans l'exemple 7 à l'exception des conditions opératoires de la mise en contact avec la solution d'octaméthylcyclotétrasiloxane dans l'heptane. L'autoclave est pressurisé sous 30 bar (3 MPa) d'hydrogène, et porté à la température de 180°C. Cette température est maintenue pendant 17 heures, sous agitation (1000 tours/min). Le catalyseur ainsi obtenu est nommé catalyseur I. Il est directement utilisé dans le test d'hydrogénation décrit dans l'exemple 12.

**Exemple 10 (comparaison) : préparation d'un catalyseur J par mise en contact avec une solution de diméthoxydiméthylsiloxane dans l'heptane**

Le catalyseur A, sous forme métallique, décrit à l'exemple 1, est introduit sans aucun contact avec l'air dans l'autoclave utilisé pour le test d'hydrogénation dans 140 mL d'heptane. Une quantité de 4.7 mL de diméthoxydiméthyl-siloxane ($Si(CH_3)_2(OCH_3)_2$, n° CAS 1112-39-6) est introduite dans l'heptane, de façon à obtenir une concentration en Si égale à 1 %poids environ. L'autoclave est ensuite fermé, purgé, puis pressurisé sous 10 bar (1 MPa) d'hydrogène, et porté à la température de 50°C. Cette température est maintenue pendant 5 heures, sous agitation (1600 tours/min). Le catalyseur ainsi obtenu est nommé catalyseur J. Il est directement utilisé dans le test d'hydrogénation décrit dans l'exemple 12.

**Exemple 11 (comparaison) : préparation d'un catalyseur K par mise en contact avec une solution de hexaméthyldisilane dans l'heptane**

Le catalyseur A, sous forme métallique, décrit à l'exemple 1, est introduit sans aucun contact avec l'air dans l'autoclave utilisé pour le test d'hydrogénation dans 140 mL d'heptane. Une quantité de 0.8 mL de hexaméthyldisilane ($Si_2(CH_3)_6$, n° CAS 1450-14-2) est introduite dans l'heptane, de façon à obtenir une concentration en Si égale à 0.2 %poids environ. L'autoclave est ensuite fermé, purgé, puis pressurisé sous 10 bar (1 MPa) d'hydrogène, et porté à la température de 50°C. Cette température est maintenue pendant 5 heures, sous agitation (1600 tours/min). Le catalyseur ainsi obtenu est nommé catalyseur K. Il est directement utilisé dans le test d'hydrogénation décrit dans l'exemple 12.

**Exemple 12 (selon l'invention) : Test catalytique en hydrogénation du 1,3-butadiène**

Les propriétés catalytiques des catalyseurs préparés selon les exemples ci-dessus sont évaluées successivement dans un procédé d'hydrogénation du 1,3-butadiène. L'hydrogénation sélective du 1,3-butadiène conduit à un mélange des trois isomères du butène (1-butène, cis-2-butène et trans-2-butène), cette hydrogénation constituant la réaction désirée. L'hydrogénation totale du 1,3-butadiène conduit au butane, lequel est produit par une réaction successive indésirable.

L'hydrogénation du 1,3-butadiène est réalisée dans un réacteur discontinu parfaitement agité de type « Grignard » composé d'un autoclave de 250 mL en acier inoxydable, muni d'une agitation mécanique. L'hydrogénation est réalisée en phase liquide, sous une pression constante de 10 bar (1 MPa) d'hydrogène, à une température de 17°C et sous une agitation de 1600 tours/min. Le solvant est l'heptane (140 mL) et la charge est constituée de 7 g de 1,3-butadiène. La consommation d'hydrogène est suivie au cours du temps par la perte de pression dans une bouteille réservoir située en amont du réacteur. Les produits de la réaction sont analysés par chromatographie en phase gazeuse.

L'activité catalytique est exprimée en moles de $H_2$ consommées par seconde et par mole de Pd. L'activité résiduelle est définie comme le rapport de l'activité du catalyseur préparé par mise en contact avec une solution contenant un composé silicé sur l'activité du catalyseur de référence. Une activité résiduelle supérieure à 100 indique que le catalyseur étudié est plus actif que le catalyseur de référence. Au contraire, une activité résiduelle inférieure à 100 indique qu'il est moins actif que le catalyseur de référence. Les activités résiduelles des catalyseurs préparés selon les exemples ci-dessus sont reportées dans le tableau 1.

Tableau 1 : Activités résiduelles des catalyseurs préparés selon les exemples ci-dessus

| Catalyseur | Composé silicé utilisé | Activité résiduelle (%) |
|---|---|---|
| A (référence) | --- | 100 |
| B | triéthylsilane - 1 %poids Si | 128 |
| C | triéthylsilane - 3 %poids Si | 222 |
| D | triéthylsilanol - 1 %poids Si | 126 |
| E | triéthylsilanol - 3 %poids Si | 186 |
| F | triéthylsilanol - 5 %poids Si | 144 |
| G | octaméthylcyclotétrasiloxane - 50 °C | 149 |
| H | octaméthylcyclotétrasiloxane - 70 °C | 248 |
| I | octaméthylcyclotétrasiloxane - 180 °C | 176 |
| J | diméthoxydiméthylsiloxane | 71 |
| K | hexaméthyldisilane | 97 |

Les catalyseurs B et C préparés par mise en contact avec une solution de triéthylsilane dans l'heptane sont donc plus actifs que le catalyseur de référence. Les catalyseurs D, E et F préparés par mise en contact avec une solution de triéthylsilanol dans l'heptane sont également plus actifs que le catalyseur de référence. Les catalyseurs G, H et I préparés par mise en contact avec une solution de octaméthylcyclotétrasiloxane dans l'heptane sont aussi plus actifs que le catalyseur de référence.

Par contre, les catalyseurs J et K préparés par mise en contact avec respectivement une solution de diméthoxydiméthylsiloxane (un siloxane non cyclique) dans l'heptane et une solution de hexaméthyldisilane (un silane ne contenant pas de liaison Si-H) dans l'heptane sont moins actifs que le catalyseur de référence.

Ces résultats mettent en évidence que toutes les molécules silicées ne permettent pas d'améliorer l'activité du catalyseur.

La sélectivité vers la formation du butène est définie à partir des teneurs dans le milieu réactionnel des différents produits de la réaction, de la façon suivante :

$$sélectivite = \frac{1But + cis2But + trans2But}{1But + cis2But + trans2But + nC4}$$

avec 1 But, la teneur massique en 1-butène,
cis2But, la teneur massique en cis-2-butène,
trans2But, la teneur massique en trans-2-butène
nC4, la teneur massique en butane

La sélectivité est d'autant plus élevée que la formation du butane est faible.

La sélectivité est évaluée pour une conversion du 1,3-butadiène de 99 % (la conversion est définie comme le rapport entre la quantité de 1,3-butadiène ayant réagi sur la quantité initiale de 1,3-butadiène). Les sélectivités des catalyseurs préparés selon les exemples ci-dessus sont reportées dans le tableau 2.

Tableau 2 : Sélectivités des catalyseurs préparés selon les exemples ci-dessus

| Catalyseur | Composé silicé utilisé | Sélectivité (%) |
| --- | --- | --- |
| A (référence) | --- | 85.5 |
| C | triéthylsilane - 3 %poids Si | 86 |
| E | triéthylsilanol - 3 %poids Si | 94 |
| H | octaméthylcyclotétrasiloxane - 70 °C | 86 |
| I | octaméthylcyclotétrasiloxane - 180 °C | 88 |

Les catalyseurs C et H préparés par mise en contact avec respectivement une solution de triéthylsilane dans l'heptane et une solution de octaméthylcyclotétrasiloxane dans l'heptane présentent une sélectivité vers la formation du butène très légèrement supérieure à celle du catalyseur de référence, alors qu'ils sont beaucoup plus actifs que le catalyseur de référence (cf. tableau 1). Cette sélectivité est encore améliorée avec le catalyseur I préparé par mise en contact à 180°C avec une solution d'octaméthylcyclotétrasiloxane dans l'heptane, puis avec le catalyseur E préparé par mise en contact avec une solution de triéthylsilanol dans l'heptane, les deux catalyseurs I et E étant également beaucoup plus actifs que le catalyseur de référence (cf. tableau 1).

**Revendications**

1. Procédé de préparation d'un catalyseur d'hydrogénation sélective dans lequel on fournit un précurseur de catalyseur comprenant au moins un métal du groupe VIII sous forme métallique et au moins un support formé d'au moins un oxyde, ledit métal du groupe VIII étant le palladium et ledit support étant une alumine, **caractérisé par le fait qu'**on effectue une étape de mise en contact dudit précurseur de catalyseur sous forme métallique en phase liquide et en présence d'une atmosphère réductrice et/ou inerte, avec un solvant apolaire contenant un composé silicé, ledit composé silicé est choisi parmi

   - les silanes contenant au moins une liaison Si-H et au moins une liaison Si-C répondant à la formule $Si_xH_yR_z$, dans laquelle x est un nombre entier compris entre 1 et 6, y est un nombre entier compris entre 1 et 2x+1 et z est un nombre entier compris entre 1 et 2x+1, la somme de y+z étant de 2x+2, le radical R est un radical hydrocarboné monovalent, identique ou différent pour chaque valence, et peut être choisi parmi un radical aliphatique saturé, un radical aliphatique non saturé, un radical cycloalkyle, un radical aryle et un radical arylalkyle,
   - les silanols répondant à la formule $Si_x(OH)R_{2x+1}$, dans laquelle x est un nombre entier compris entre 1 et 6

et R est un radical tel que défini ci-dessus,
- et les siloxanes cycliques dans lequel la chaîne principale -Si-O-Si-O- forme un cycle et est composée d'unité $(R_2SiO)_n$, avec n étant un nombre entier compris entre 3 à 11, et R est un radical tel que défini ci-dessus.

2. Procédé selon la revendication 1, dans lequel le solvant apolaire est choisi parmi un solvant hydrocarboné aliphatique, un solvant hydrocarboné cyclique, un solvant hydrocarboné aromatique, une essence partiellement saturée, un effluent partiellement hydrogéné issu d'un procédé d'hydrogénation sélective, ou un mélange de ces solvants.

3. Procédé selon l'une des revendications 1 ou 2, dans lequel le composé silicé est présent dans le solvant apolaire dans une concentration entre 0,01 et 10 % pds de silicium.

4. Procédé selon l'une des revendications 1 à 3, dans lequel l'étape de mise en contact est effectuée à une température comprise entre 20 et 200°C.

5. Procédé selon l'une des revendications 1 à 4, lequel comprend en outre les étapes suivantes, lesdites étapes étant effectuées avant l'étape de mise en contact :

   a) au moins une étape, dans laquelle on imprègne une solution contenant au moins un précurseur dudit métal du groupe VIII, sur ledit support,
   b) au moins une étape dans laquelle on sèche le support imprégné issu de l'étape a),
   c) au moins une étape, dans laquelle on calcine le support séché issu de l'étape b) de manière à obtenir au moins ledit métal dudit groupe VIII sous forme oxyde,
   d) au moins une étape, dans laquelle on effectue un traitement réducteur du support calciné issu de l'étape c) par mise en contact avec un gaz réducteur de manière à obtenir au moins ledit métal dudit groupe VIII sous forme métallique.

6. Procédé selon la revendication 5, dans lequel l'étape b) de séchage est réalisée à une température comprise entre 20 et 160°C, l'étape c) de calcination est réalisée à une température comprise entre 150 et 800°C et l'étape d) de traitement réducteur par mise en contact avec un gaz réducteur est effectuée à une température supérieure ou égale à 50°C.

7. Procédé selon l'une des revendications 5 ou 6 comprenant en outre une étape e) dans laquelle on effectue une passivation du précurseur de catalyseur par un composé soufré, ladite étape de passivation est effectuée soit après l'étape c) de calcination et avant l'étape d) de traitement réducteur du précurseur de catalyseur, soit après l'étape d) de traitement réducteur du précurseur de catalyseur, soit après l'étape de mise en contact.

**Patentansprüche**

1. Verfahren zur Herstellung eines Katalysators zur selektiven Hydrierung, bei dem ein Katalysatorvorläufer bereitstellt wird, umfassend mindestens ein Metall der Gruppe VIII in metallischer Form und mindestens einen Träger, der von mindestens einem Oxid gebildet ist, wobei das Metall der Gruppe VIII Palladium ist und der Träger ein Aluminiumoxid ist, **dadurch gekennzeichnet, dass** ein Schritt des Inkontaktbringens des Katalysatorvorläufers in metallischer Form in flüssiger Phase und im Beisein einer reduzierenden und/oder inerten Atmosphäre mit einem apolaren Lösungsmittel, das eine siliziumhaltige Verbindung enthält, durchgeführt wird, wobei die siliziumhaltige Verbindung ausgewählt ist unter

   - den Silanen, die mindestens eine Si-H-Verbindung und mindestens eine Si-C-Verbindung enthalten, entsprechend der Formel $Si_xH_yR_z$, wobei x eine ganze Zahl zwischen 1 und 6, y eine ganze Zahl zwischen 1 und 2x+1 ist, die Gruppe R eine monovalente Kohlenwasserstoffgruppe ist, die für jede Valenz identisch oder unterschiedlich ist und unter einer gesättigten aliphatischen Gruppe, einer nicht gesättigten aliphatischen Gruppe, einer Zykloalkylgruppe, einer Arylgruppe und einer Arylalkylgruppe ausgewählt sein kann,
   - denen Silanolen entsprechend der Formel $Si_x(OH)R_{2x+1}$, wobei x eine ganze Zahl zwischen 1 und 6 und R eine Gruppe, wie oben definiert, ist,
   - und den zyklischen Siloxanen, wobei die Hauptkette -Si-O-Si-O- einen Zyklus bildet und von einer Einheit $(R_2SiO)_n$ gebildet ist, wobei n eine ganze Zahl zwischen 3 bis 11 und R eine Gruppe, wie oben definiert, ist.

2. Verfahren nach Anspruch 1, bei dem das apolare Lösungsmittel ausgewählt ist unter einem aliphatischen Kohlenwasserstofflösungsmittel, einem zyklischen Kohlenwasserstofflösungsmittel, einem aromatischen Kohlenwasserstofflösungsmittel, eine teilweise gesätigten Benzin, einem teilweise hydrogenierten Effluent, das von einem Verfahren zur selektiven Hydrierung stammt, oder einer Mischung dieser Lösungsmittel.

3. Verfahren nach einem der Ansprüche 1 oder 2, bei dem die siliziumhaltige Verbindung in dem apolaren Lösungsmittel in einer Konzentration zwischen 0,01 und 10 Gew.-% Silizium vorhanden ist.

4. Verfahren nach einem der Ansprüche 1 bis 3, bei dem der Schritt des Inkontaktbringens bei einer Temperatur zwischen 20 und 200°C erfolgt.

5. Verfahren nach einem der Ansprüche 1 bis 4, das ferner die folgenden Schritte umfasst, wobei die Schritte vor dem Schritt des Inkontaktbringens durchgeführt werden:

   a) mindestens einen Schritt, in dem eine Lösung, die mindestens einen Vorläufer des Metalls der Gruppe VIII enthält, auf dem Träger imprägniert wird,
   b) mindestens einen Schritt, in dem der imprägnierte Träger aus Schritt a) getrocknet wird,
   c) mindestens einen Schritt, in dem der getrocknete Träger aus Schritt b) kalziniert wird, um mindestens das Metall der Gruppe VIII in Oxidform zu erhalten,
   d) mindestens einen Schritt, in dem eine Reduktionsbehandlung des kalzinierten Trägers aus Schritt c) durch Inkontaktbringen mit einem Reduktionsgas durchgeführt wird, um mindestens das Metall der Gruppe VIII in metallischer Form zu erhalten.

6. Verfahren nach Anspruch 5, bei dem der Schritt b) des Trocknens bei einer Temperatur zwischen 20 und 160°C erfolgt, der Schritt c) des Kalzinierens bei einer Temperatur zwischen 150 und 800°C erfolgt, und der Schritt d) der Reduktionsbehandlung durch Inkontaktbringen mit einem Reduktionsgas bei einer Temperatur größer oder gleich 50°C erfolgt.

7. Verfahren nach einem der Ansprüche 5 oder 6, ferner umfassend einen Schritt e), in dem eine Passivierung des Katalysatorvorläufers durch eine schwefelhaltige Verbindung durchgeführt wird, wobei der Passivierungsschritt entweder nach dem Schritt d) der Kalzinierung und vor dem Schritt d) der Reduktionsbehandlung des Katalysatorvorläufers erfolgt, oder nach dem Schritt d) der Reduktionsbehandlung des Katalysatorvorläufers oder nach dem Schritt des Inkontaktbringens.

**Claims**

1. Process for preparing a selective hydrogenation catalyst, wherein is provided a catalyst precursor, comprising at least one group VIII metal in the metallic form, and at least one support formed of at least one oxide, said group VIII metal being palladium and said support being alumina, **characterised in that** a step is performed of contacting the said catalyst precursor in the metallic form, in the liquid phase and in the presence of a reducing and/or inert atmosphere, with a non-polar solvent containing a silicon compound, the said silicon compound is selected from

   - the silanes containing at least one Si-H bond and at least one Si-C bond corresponding to the formula $Si_xH_yR_z$, wherein x is an integer within the range 1 to 6, y is an integer within the range 1 to 2x+1, and z is an integer within the range 1 to 2x+1, the sum of y+z being 2x+2, the R radical is a monovalent hydrocarbon radical, identical or different for each valence, and may be selected from a saturated aliphatic radical, an unsaturated aliphatic radical, a cycloalkyl radical, an aryl radical and an arylalkyl radical,
   - the silanols corresponding to the formula $Si_x(OH)R_{2x+1}$, wherein x is an integer within the range 1 to 6 and R is a radical as defined above,
   - and the cyclic siloxanes in which the principal -Si-O-Si-O- chain forms a ring and is composed of $(R_2SiO)_n$ units, with n being an integer within the range 3 to 11 and R a radical as defined above.

2. A process according to claim 1, wherein the non-polar solvent is selected from an aliphatic hydrocarbon solvent, a cyclic hydrocarbon solvent, an aromatic hydrocarbon solvent, a partially saturated gasoline, a partially hydrogenated effluent derived from a selective hydrogenation process, or a mixture of these solvents.

3. A process according to any one of claims 1 or 2, wherein the silicon compound is present in the non-polar solvent in a concentration between 0.01 and 10 wt.% of silicon.

4. A process according to any one of claims 1 to 3, wherein the contacting step is performed at a temperature within the range 20 to 200°C.

5. A process according to any one of claims 1 to 4, which further comprises the following steps, the said steps being performed before the contacting step:

   a) at least one step in which a solution containing at least one precursor of the said group VIII metal is impregnated onto the said support,
   b) at least one step in which the impregnated support resulting from step a) is dried,
   c) at least one step in which the dried support resulting from step b) is calcined, so as to obtain at least the said metal from the said group VIII in oxide form,
   d) at least one step in which a reductive treatment of the calcined support resulting from step c) is performed by contact with a reducing gas so as to obtain at least the said metal of the said group VIII in the metallic form.

6. A process according to claim 5, wherein drying step b) is performed at a temperature within the range 20 to 160°C, calcining step c) is performed at a temperature within the range 150 to 800°C and step d) of reductive treatment by contact with a reducing gas is performed at a temperature above or equal to 50°C.

7. A process according to any one of claims 5 or 6 further comprising a step e), wherein a passivation of the catalyst precursor is performed by a sulphur compound, the said passivation step is performed either after calcination step c) and before step d) of reductive treatment of the catalyst precursor, or after step d) of reductive treatment of the catalyst precursor, or after the contact step.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20060229478 A
- WO 9511753 A
- EP 955089 A
- US 20120071700 A
- FR 2922784

- EP 0466567 A
- US 5153163 A
- FR 2676184
- WO 2004098774 A
- EP 0707890 A

**Littérature non-brevet citée dans la description**

- **A. MOLNAR ; I. BUCSI ; M. BARTOK ; F. NOTHEISZ ; G.V. SMITH.** Reactions of Organosilicon Compounds on Metals : III. Selective Poisoning by Et3SiH of Catalytic Hydrogenation and Dehydrogenation. *Journal of Catalysis,* 1986, vol. 98, 386
- **G.V. SMITH ; S. TJANDRA ; M. MUSOIU ; T. WILTOWSKI ; F. NOTHEISZ ; M. BARTÓK ; I. HANNUS ; D. OSTGARD ; V. MALHOTRA.** Modified Activities and Selectivities of Silated-oxidized-Reduced Pd and Pt catalysts. *Journal of Catalysis,* 1996, vol. 161, 441

- **E.W. SHIN ; C.H. CHOI ; K.S. CHANG ; Y.H. NA ; S.H. MOON.** Properties of Si-modified Pd catalyst for selective hydrogenation of acetylene. *Catalysis Today,* 1998, vol. 44, 137
- **W.J. KIM ; E. W. SHIN ; J. H. KANG ; S. H. MOON.** Performance of Si-modified Pd catalyst in acetylene hydrogenation: catalyst deactivation behavior. *Applied Catalysis A: General,* 2003, vol. 251, 305